# EUROPEAN PATENT APPLICATION

(11) **EP 2 361 639 A2**
(43) Date of publication of application: **31.08.2011**
(21) Application number: 11167269.7
(22) Date of filing: 27.03.2008
(51) Int. Cl.: A61L 9/20

(54) **Photocatalytic air treatment system and method**

(30) Priority: 27.03.2007 US 728949
(62) Divisional of application: 08153442.2
(71) Applicant: Hayman Jr., John J., Marietta, Georgia 30062 (US)
(72) Inventor: Hayman Jr., John J., Marietta, Georgia 30062 (US)
(74) Representative: Walsh, Marie Goretti

(57) **Abstract**

A photocatalytic air treatment system, including apparatuses and methods, for killing and/or mineralizing bacteria, viruses, mold, fungi, spores, mycotoxins, allergens, and other similar microorganisms or agents, and for oxidizing volatile organic compounds (VOCs). The system comprises one or more reactor beds configured in one or more stages with each reactor bed including a plurality of photocatalyst coated media substantially surrounding a plurality of sheathed ultraviolet light sources that may be arranged in a plurality of configurations. Adjacent ultraviolet light sources are positioned so as to create killing zones of photocatalyst coated media therebetween that are irradiated with ultraviolet light from multiple sources and in which an increased number of hydroxyl radicals are present. The photocatalyst generally comprises titanium dioxide, but may include one or more enhancers. The media is formed from or is coated with a material that induces the photocatalyst to form a nano-particle structure.

## Description

### CROSS REFERENCE TO RELATED APPLICATION

This application is based on and claims the benefit of priority of U.S. patent application Ser. No. 11/728,949 entitled "Photocatalytic Air Treatment System and Method" filed on Mar. 27, 2007.

### FIELD OF THE INVENTION

The present invention relates, generally, to the field of air treatment systems and, more specifically, to air treatment systems and methods using a photocatalytic reaction to kill and/or mineralize bacteria, viruses, mold, fungi, spores, mycotoxins, allergens, and other similar microorganisms or agents and to oxidize volatile organic compounds (VOCs).

### BACKGROUND OF THE INVENTION

For many years, people have suffered adverse physical effects including infections and allergic reactions that have been knowingly or unknowingly caused by or related to exposure to bacteria, viruses, mold, fungi, spores, mycotoxins, allergens, and other similar microorganisms or agents. In attempts to reduce the suffering caused by such microorganisms or agents, the medical community and pharmaceutical companies have directed substantial resources toward developing various drugs and other forms of medical treatments, but without overwhelming success. In other attempts to reduce the suffering caused by such microorganisms or agents, a number of inventors and manufacturers have developed various devices that are targeted at killing them.

Some such devices, including those sometimes found in physician's offices, hospitals, or other medical facilities utilize ultraviolet light to treat air through which the ultraviolet light passes. Unfortunately, such ultraviolet light devices have not been overly successful at least in part due to their reliance on sufficiently heating the microorganisms or agents as a mechanism to kill them. In many such ultraviolet light devices, treatment of air is attempted by allowing the air to move by natural or forced convection through ultraviolet light produced by ultraviolet bulbs. By virtue of the microorganisms or agents being entrained in the air, they also pass through and are struck by photons of ultraviolet light that heats them and, potentially, kills them. However, because the exposure time of the microorganisms or agents to sufficiently intense ultraviolet light is often too short in duration due to movement of the air, many of the microorganisms or agents escape without being hit by a sufficient number of photons to heat them enough to kill them.

Other devices that have been developed produce ozone that may, in turn, react with various compounds found in carpets, rugs, and other items to create formaldehyde. Also, the ozone may create hydroxyl radicals that attack organic compounds such as those found in the breathing passages and tissues of humans and animals.

Therefore, there exists in the industry, a need for a system, including apparatuses and methods, for treating air to kill and/or mineralize various microorganisms or agents and to oxidize volatile organic compounds, and that addresses the above-described, and other, problems, difficulties, and/or shortcomings of current systems.

### SUMMARY OF THE INVENTION

The invention is defined in the appended Claims 1 to 26. Broadly described, the present invention comprises a photocatalytic air treatment system, including apparatuses and methods, for killing and/or mineralizing bacteria, viruses, mold, fungi, spores, mycotoxins, allergens, and other similar microorganisms or agents, and for oxidizing volatile organic compounds (VOCs). More particularly, the present invention comprises a photocatalytic air treatment system, including apparatuses and methods, that utilizes ultraviolet light in connection with a photocatalytic reaction that causes the heating, oxidation, and/or mineralization of bacteria, viruses, mold, fungi, spores, mycotoxins, allergens, other similar microorganisms or agents, and volatile organic compounds (VOCs).

In exemplary embodiments, the photocatalytic air treatment system of the present invention comprises one or more reactor beds configured in one or more stages with each reactor bed including a plurality of photocatalyst coated media therein formed from substrate media coated with a photocatalyst substance and, sometimes, also with a reaction enhancing substance. The substrate media are formed, for example and not limitation, from a glass or glass-like material that is non-reactive with the photocatalyst substance and the reaction enhancing substance, and that induces the photocatalyst substance to form on each substrate media as a nano-particle structure rather than as a mere closely packed layer, thereby enabling and causing the photocatalyst substance to be struck by photons of ultraviolet light from a variety of directions. Each reactor bed also includes a plurality of ultraviolet light sources located therewithin that are substantially surrounded by photocatalyst coated media such that photons of ultraviolet light emitted by the ultraviolet light sources are directed outwardly at the photocatalyst coated media. The ultraviolet light sources are, generally, located in one or more arrangements at positions relative to one another that create one or more volumes of photocatalyst coated media in which ultraviolet light produced by multiple ultraviolet light sources is incident thereon and in which the irradiance of the ultraviolet light contributed to the volumes by multiple ultraviolet light sources and incident on the photocatalyst coated media is at a maximum. Each reactor bed additionally includes a plurality of sheaths cooperatively located with the plurality of ultraviolet light sources such that at least one sheath is intermediate an ultraviolet light source and surrounding photocatalyst coated media. The photocatalytic air treatment system further comprises an air-handling unit that forces or induces air to flow through at least one reactor bed. In some exemplary embodiments, the photocatalytic air treatment system still further comprises a heating unit that heats the air being treated in order to reduce the humidity thereof.

Advantageously, the photocatalytic air treatment system's use of photocatalyst coated media in which the photocatalyst substance forms a nano-particle structure thereon dramatically increases the number of reaction sites available for bacteria, viruses, mold, fungi, spores, mycotoxins, allergens, other similar microorganisms or agents, and volatile organic compounds (VOCs) to undergo an oxidation reaction with a hydroxyl radical (OH⁻) produced as part of the photocatalyic reaction. By dramatically increasing the number of available reaction sites and increasing the number of such oxidation reactions that occur, the system's ability to treat air, to kill and/or mineralize bacteria, viruses, mold, fungi, spores, mycotoxins, allergens, and other similar microorganisms or agents, and to oxidize volatile organic compounds (VOCs) is greatly improved over other systems. The system's sometime use of a reaction enhancing substance also coated on the substrate media improves the reactivity of the photocatalyst substance, thereby increasing the number of hydroxyl radicals (OH⁻) available for and that actually react in an oxidation reaction with such microorganisms or agents and with volatile organic compounds (VOCS) and enhancing the system's ability to improve the quality of the air that it treats.

Also advantageously, the volumes of photocatalyst coated media created by the photocatalytic air treatment system's arrangement of the ultraviolet light sources relative to one another have an increased level of photocatalytic reactivity due to the irradiance of the ultraviolet light incident on the photocatalyst coated media thereof being at a maximum. Because the volumes have an increased level of photocatalytic reactivity, the volumes also have an increased number of hydroxyl radicals (OH⁻) available for and that actually react in an oxidation reaction with bacteria, viruses, mold, fungi, spores, mycotoxins, allergens, other similar microorganisms or agents, and volatile organic compounds (VOCs) entrained in the untreated air, thereby improving the system's ability to kill, mineralize, or oxidize such microorganisms, agents, and volatile organic compounds (VOCs). Additionally, the heating and reduction of humidity of the untreated air with a heating unit in some exemplary embodiments also increases the level of photocatalytic reactivity and the production of hydroxyl radicals (OH⁻) with a similar effect on the system's ability to kill, mineralize, or oxidize such microorganisms, agents, and volatile organic compounds (VOCs).

Further advantageously, the photocatalytic air treatment system's inclusion of a sheath for each ultraviolet light source in a reactor bed enables the ultraviolet light sources to be replaced without disturbing the photocatalyst coated media also present in the reactor bed. In addition, the system's ability to include one or more reactor beds in a particular configuration thereof allows customization of the system to produce desired levels of air treatment.

Other advantages and benefits of the present invention will become apparent upon reading and understanding the present specification when taken in conjunction with the appended drawings.

### BRIEF DESCRIPTION OF THE DRAWINGS

FIG. 1 displays a schematic, top plan view of a photocatalytic air treatment system for treating air in accordance with a first exemplary embodiment of the present invention.

FIG. 2 displays a schematic, sectional view of the reactor bed of the photocatalytic air treatment system of FIG. 1 taken along lines 2-2.

FIG. 3 displays a schematic, partial sectional view of the reactor bed of the photocatalytic air treatment system of FIG. 1 taken along lines 3-3.

FIG. 4 displays a schematic, top plan view of a photocatalytic air treatment system for treating air in accordance with a second exemplary embodiment of the present invention.

FIG. 5 displays a schematic, sectional view of the reactor bed of the photocatalytic air treatment system of FIG. 4 taken along lines 5-5.

FIG. 6 displays a schematic, top plan view of a reactor bed of a photocatalytic air treatment system for treating air in accordance with a third exemplary embodiment of the present invention.

FIG. 7 displays a schematic, sectional view of the reactor bed of the photocatalytic air treatment system of FIG. 6 taken along lines 7-7.

FIG. 8 displays a schematic, top plan view of a photocatalytic air treatment system for treating air in accordance with a fourth exemplary embodiment of the present invention.

FIG. 9 displays a schematic, top plan view of a photocatalytic air treatment system for treating air, in accordance with a fifth exemplary embodiment of the present invention, having multiple stages of air treatment.

### DETAILED DESCRIPTION OF THE INVENTION

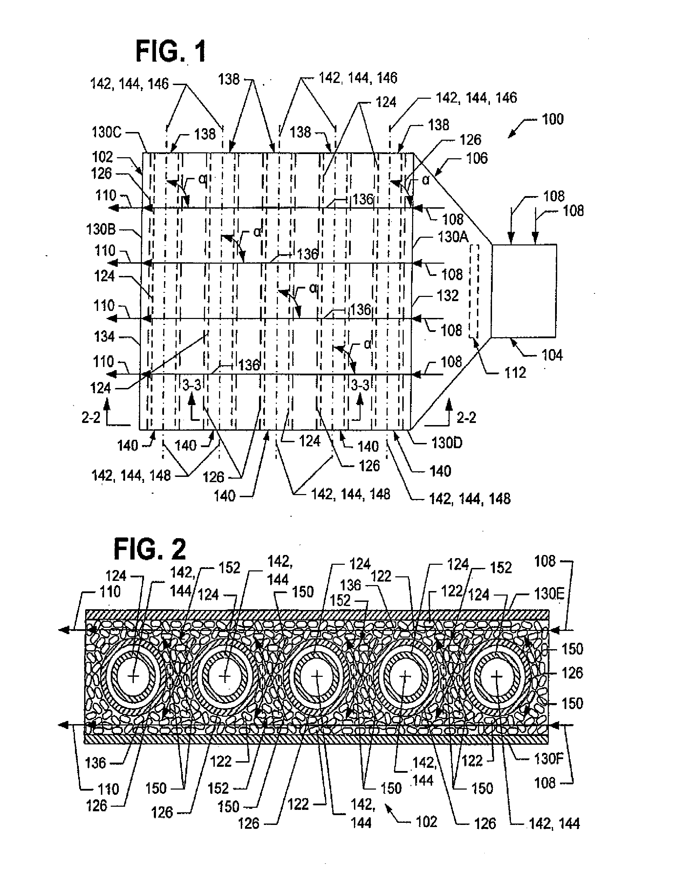

Referring now to the drawings in which like numerals represent like elements or steps throughout the several views, FIG. 1 displays a schematic, top plan view of a photocatalytic air treatment system 100, according to a first exemplary embodiment of the present invention, for treating air by killing and/or mineralizing bacteria, viruses, mold, fungi, spores, mycotoxins, allergens, and other similar microorganisms or agents, and for oxidizing volatile organic compounds (VOCs), that may be present therein. The photocatalytic air treatment system 100 comprises a reactor bed 102, an air-handling unit 104, and a transition member 106 interposed between and connected to the reactor bed 102 and the air-handling unit 104. The air-handling unit 104 is adapted to pull untreated air 108 (i.e., indicated by arrows 108) from the environment in which the photocatalytic air treatment system 100 is present or from another source and to direct the untreated air 108 into the reactor bed 102 via the transition member 106. Generally, the air-handling unit 104 comprises a fan or blower that directs, or blows, untreated air 108 into the reactor bed 102 at a static pressure and volumetric flow rate selected and sufficient to overcome the static pressure drop caused by the reactor bed 102 during flow therethrough, to produce a desired volumetric rate of treated air 110 (i.e., indicated by arrows 110) exiting the system 100, and to achieve a desired quality of air treatment as determined by measuring (e.g., in parts per million (ppm) or parts per billion (ppb)) the quantity of killed, mineralized, or oxidized bacteria, viruses, mold, fungi, spores, mycotoxins, allergens, other similar microorganisms or agents, and volatile organic compounds (VOCs) present in the treated air 110 relative to the quantity of the same present in the untreated air 108. The transition member 106 is configured to direct untreated air 108 exiting the air-handling unit 104 into the reactor bed 102 for treatment. Generally, the transition member 106 comprises a plenum, duct, or similar structure configured to direct and distribute the untreated air 108 into the reactor bed 102 in a manner that provides a substantially equal flow of untreated air 108 to all parts of the reactor bed 102. It should be noted that in some embodiments of the present invention, the air-handling unit 104 is connected directly to the reactor bed 102 absent any transition member 106 therebetween. It should also be noted that in some embodiments of the present invention, the air-handling unit 104 may be positioned relative to the reactor bed 102 and operated in a manner that induces a flow of untreated air 108 through the reactor bed 102 instead of forcing a flow of untreated air 108 through the reactor bed 102.

The photocatalytic air treatment system 100 may further comprise, as illustrated in FIG. 1, a heating element 112 adapted to heat the untreated air 108 prior to its entrance into the reactor bed 102. Generally, the heating element 112 comprises an electric resistance heater, heating strip, or other suitable device for raising the temperature of the untreated air 108 above its temperature when entering the air-handling unit 104. By raising the temperature of the untreated air 108 before it enters the reactor bed 102, the reaction rate of the photocatalytic reaction (described in more detail below) is increased and the production of hydroxyl radicals (OH⁻) is also increased, thereby enhancing the probability that bacteria, viruses, mold, fungi, spores, mycotoxins, allergens, other similar microorganisms or agents, and/or volatile organic compounds (VOCs) will come into contact with a hydroxyl radical (OH⁻) and undergo an oxidation reaction that kills, mineralizes, or destroys same, as the case may be. Further, by raising the temperature of the untreated air 108, the humidity level of the untreated air 108, or relative humidity, is reduced and the reaction rate of the photocatalytic reaction is increased with similar effects resulting as those due to the increase in temperature of the untreated air 108. It should be noted, however, that while the inclusion and operation of a heating element 112 is beneficial to the system's overall quality of air treatment, the photocatalytic air treatment system 100 need not include a heating element 112 in order to achieve substantial reductions in the quantities of volatile organic compounds (VOCs) and/or live, reproducible bacteria, viruses, mold, fungi, spores, mycotoxins, allergens, and other similar microorganisms or agents present in the treated air 110 exiting the system 100.

According to the present embodiment, the reactor bed 102 comprises an enclosure 120 and a plurality of photocatalyst coated media 122 that are contained by and within the enclosure 120. Notably, the photocatalyst coated media 122 are arranged within the enclosure 120 in a substantially random manner such that air passing through the reactor bed 102 collides with, or comes into contact with, many photocatalyst coated media 122, thereby increasing the amount of time that bacteria, viruses, mold, fungi, spores, mycotoxins, allergens, other similar microorganisms or agents, and volatile organic compounds (VOCs) present in the untreated air 108 are in contact with the photocatalyst coated media 122 and increasing the system's ability to kill, mineralize, or oxidizing the same. The photocatalyst coated media 122 are also arranged within the enclosure 120 so as to increase the number of photons of ultraviolet light that strike them. Each photocatalyst coated media 122 includes a substrate media that is coated, at least in part, with a photocatalyst substance on the surface thereof that undergoes a photocatalytic reaction when exposed to ultraviolet light. The photocatalytic reaction produces hydroxyl radicals (OH⁻) on the surface of the substrate media that are available to combine, in an oxidation reaction, with bacteria, viruses, mold, fungi, spores, mycotoxins, allergens, other similar microorganisms or agents, and volatile organic compounds (VOCs) present in the untreated air 108. Generally, in the exemplary embodiments described herein, the photocatalyst substance comprises titanium dioxide (TiO₂), but may also comprise other substances alone or in combination with the titanium dioxide (TiO₂) in other embodiments. Thus, the photocatalyst substance may further comprise an enhancing substance (sometimes referred to herein as an "enhancer") that increases the reaction rate of the photocatalytic reaction, thereby causing the production of an increased number hydroxyl radicals available for an oxidation reaction as described above. In the exemplary embodiments described herein, the photocatalyst substance may further comprise an enhancer including zirconium dioxide (ZrO₂) in a quantity of about ten percent (10%) of the total photocatalyst substance. It should be noted, however, that the scope of the present invention includes the incorporation and use of enhancing substances other than zirconium dioxide (ZrO₂).

The substrate media of the plurality of photocatalyst coated media 122 are selected to have shapes or forms providing a substantial amount of surface area for coating with a photocatalyst substance and for supplying sites for hydroxyl radicals (OH⁻) to be exposed to untreated air 108 flowing through the reactor bed 102. By utilizing substrate media having shapes that provide maximal surface area, the amount of photocatalyst substance that is applied to and present on the substrate media is increased relative to the amount that may be applied to other shapes with complementary increases occurring in the number of hydroxyl radicals (OH⁻) that are created by the photocatalytic reaction of the photocatalyst substance with ultraviolet light and in the number of hydroxyl radicals (OH⁻) that are actually contacted by and undergo an oxidizing reaction with bacteria, viruses, mold, fungi, spores, mycotoxins, allergens, other similar microorganisms or agents, and volatile organic compounds (VOCs) present in the untreated air 108. In accordance with the exemplary embodiments described herein, the substrate media are all generally of a tubular shape or form somewhat similar to macaroni and are coated at least partially with a photocatalyst substance on inner and outer surfaces thereof, thereby providing a substantial amount of surface area for the creation, residence, and reaction of hydroxyl radicals (OH⁻). In other exemplary embodiments of the present invention, the substrate media of the photocatalyst coated media 122 may have a cylindrical, spherical, toroidal, polyhedrical, or other shape or form. Furthermore, in other exemplary embodiments of the present invention, the substrate media of the photocatalyst coated media 122 may have a plurality of different shapes or forms.

In the exemplary embodiments of the present invention described herein, the substrate media of the plurality of photocatalyst coated media 122 are formed from a material that does not react with (e.g., is inert relative to) the photocatalyst substance applied thereto and that, perhaps, more importantly induces the applied photocatalyst substance to form a nano-particle structure atop the surface(s) of the substrate media instead of forming only a closely-packed layer. By virtue of the photocatalyst substance forming a nano-particle structure, the number of potential sites and surface area for photocatalysis to occur and for the concomitant creation of hydroxyl radicals (OH⁻) and their contact with and oxidation of bacteria, viruses, mold, fungi, spores, mycotoxins, allergens, other similar microorganisms or agents, and volatile organic compounds (VOCs) is dramatically increased over the number of potential sites and surface area that would otherwise be available for such to occur if the material of the substrate media did not induce the formation of a nano-particle structure. Generally, the material of the substrate media of the described exemplary embodiments comprises bora silica glass, but other materials capable of causing the applied photocatalyst substance to form a nano-particle structure are also considered to be within the scope of the present invention. It should also be noted that the scope of the present invention includes other materials for substrate media that are normally reactive with the photocatalyst substance applied thereto, but that are pre-coated with another substance that renders them non-reactive with the photocatalyst substance prior to coating them with the photocatalyst substance. Such reactive materials include, for example and not limitation, materials commonly classified as plastics, metals, and ceramics.

According to the present invention, the photocatalytic air treatment system 100 further comprises a plurality of ultraviolet light sources 124 that are positioned so as to emit photons of ultraviolet light at the photocatalyst coated media 122 and at the photocatalyst substance thereof, thereby causing a photocatalytic reaction of the photocatalyst substance to occur, hydroxyl radicals (OH⁻) to be generated by the photocatalytic reaction, and oxidation reactions of the hydroxyl radicals (OH⁻) and bacteria, viruses, mold, fungi, spores, mycotoxins, allergens, other similar microorganisms or agents, and volatile organic compounds (VOCs) present in the untreated air 108 to occur. The ultraviolet light sources 124 are generally adapted to produce light having one or more wavelengths entirely within the ultraviolet portion (e.g., wavelengths less than 400 nm) of the electromagnetic spectrum. However, the scope of the present invention should be understood as including ultraviolet light sources 124 that may produce other light having one or more wavelengths that are outside of, or otherwise not within, the ultraviolet portion (e.g., wavelengths greater than 400 nm) of the electromagnetic spectrum. Also, the ultraviolet light sources 124 are generally embodied in the present invention in the form of elongated, tubular, T5 lamps or bulbs that generate ultraviolet light having a wavelength within a range of about 240 nm to 260 nm with an irradiance within a range of approximately 20µW/cm² to 30µW/cm². However, it should be understood that the scope of the present invention includes ultraviolet light sources 124 that generate ultraviolet light having an irradiance greater than 1µW/cm³. According to the exemplary embodiments described herein, the ultraviolet light sources 124 are preferably adapted to produce light having one or more wavelengths within the UV-C bandwidth of the electromagnetic spectrum, but may include one or more wavelengths within the UV-A, UV-B, and/or UV-C bandwidths of the electromagnetic spectrum.

In other embodiments within the scope of the present invention, the ultraviolet light sources 124 may produce different levels of irradiance and may be embodied in other forms, including, for example and not limitation, ultraviolet lamps or bulbs having non-tubular or other shapes, and ultraviolet light emitting diodes (LEDs). In still other embodiments within the scope of the present invention, the ultraviolet light sources 124 may be replaced by other devices such as lamps or bulbs other than ultraviolet fluorescent lamps or bulbs, non-ultraviolet light emitting diodes (LEDs), waveguides that direct ultraviolet light and/or other forms of energy at the photocatalyst coated media 122, mercury vapor lamps (and, more particularly, high-pressure mercury vapor lamps), and microwave sources that are operable to produce a similar amount of energy as ultraviolet light sources 124 and/or that are operable to impart a sufficient amount of energy to the photocatalyst substance in order to cause photocatalysis and the above-described photocatalytic reaction of the photocatalyst substance to occur. It should be noted that the scope of the present invention also includes reactor beds 102 having any number of lamps and/or bulbs, lamps and/or bulbs having the same or different sizes in terms of diameter and length, lamps and/or bulbs having the same or different wattages, and/or any combination of the foregoing.

In the exemplary embodiments of the present invention described herein, the reactor bed 102 further comprises a plurality of sheaths 126 for receiving the ultraviolet light sources 124 therein and for separating the ultraviolet light sources 124 from the photocatalyst coated media 122 that substantially surround the sheaths 126 and ultraviolet light sources 124. By separating the ultraviolet light sources 124 from the photocatalyst coated media 122 with intermediate sheaths 126, the ultraviolet light sources 124 may be inserted into and removed from the reactor bed 102 (i.e., inserted and removed from the sheaths 126) without coming into contact with the photocatalyst coated media 122, thereby making replacement of the ultraviolet light sources 124 much easier and less time consuming whenever such replacement is necessary. The sheaths 126 are generally formed from a quartz, or quartz-like, material that enables ultraviolet light from the ultraviolet light sources 124 to pass therethrough substantially unaffected and that does not react with the photocatalyst substance of the photocatalyst coated media 122 in contact therewith. It should be noted that although the exemplary embodiments of the present invention described herein include a plurality of sheaths 130, the scope of the present invention includes other exemplary embodiments that do not include such sheaths 130. It should also be noted that even though the photocatalytic air treatment system 100 of the present invention is illustrated herein via different embodiments having particular numbers of ultraviolet light sources 124 and/or sheaths 126, the scope of the present invention comprises other exemplary embodiments having different numbers and arrangements of ultraviolet light sources 124 and/or sheaths 126.

The enclosure 120 of the reactor bed 102 is formed from a plurality of panels 130 that confine the photocatalyst coated media 122 within the enclosure 120 and that define a generally rectangular shape when viewed in top plan view as in FIG. 1. A first opposed pair of panels 130A, 130B respectively define an air inlet 132 and an air outlet 134 of the reactor bed 102. Panel 130A is generally formed from a perforated or mesh-like material suitable to confine the photocatalyst coated media 122 and is adapted to receive untreated air 108 from transition member 106 and to allow the received untreated air 108 to pass therethrough and into the reactor bed 102. Panel 130B is similarly formed from a perforated or mesh-like material suitable to confine the photocatalyst coated media 122 and is adapted to receive treated air 110 from the reactor bed 102 and to allow the received treated air 110 to pass therethrough and to exit the photocatalytic air treatment system 100. Second and third opposed pairs of such panels 130C, 130D, 130E, 130F are generally formed from a material that it is not air permeable and are adapted to direct untreated air 108 from the air inlet 132 and through the reactor bed 102 in a predominant, or primary, direction (e.g., designated by arrows 136) toward the air outlet 134. It should be noted that in some exemplary embodiments, the reactor bed 102 and its enclosure 120 may comprise a removable chamber, or cartridge, that may be disconnected and/or removed from fluid communication with the transition member 106 and air handling unit 104 so that it can be replaced after the elapse of an appropriate period of time (for example and not limitation, one year) with a new or reconditioned removable chamber, or cartridge, having an identical or similar reactor bed 102 and enclosure 120, and having new or refurbished ultraviolet light sources 124, sheaths 126, and/or photocatalyst coated media 122. Additionally, it should be noted that in certain other embodiments, a removable and replaceable HEPA or % HEPA all-inclusive filter(s) may be connected in fluid communication with the reactor bed 102 and its enclosure 120 (whether comprising a removable chamber or not) generally at either the intake or exhaust thereof so that the air also passes through such filter(s) to receive further treatment and conditioning. In still other embodiments, a HEPA filter or % HEPA all-inclusive filter may be integral with the reactor bed 102 and enclosure 120, thereby together defining and forming a removable and replaceable chamber or cartridge of the photocatalytic air treatment system 100. It should be noted that in other embodiments, a HEPA filter or % HEPA all-inclusive filter(s) may be positioned at various other locations within the air flow path of the photocatalytic air treatment system 100.

The sheaths 126 extend substantially between the second opposed pair of panels 130C, 130D at locations corresponding respectively to holes 138, 140 defined by panels 130C, 130D. Each sheath 126, according to the exemplary embodiments described herein, has a generally elongate sleeve-like shape with a longitudinal centerline 142 and is sized to receive a corresponding ultraviolet light source 124 therein having a longitudinal centerline 144 such that longitudinal centerlines 142, 144 are substantially collinear. The holes 138, 140 in respective panels 130C, 130D have longitudinal centerlines 146, 148 that are also substantially collinear with the longitudinal centerlines 142, 144 of the respective sheaths 126 and ultraviolet light sources 124. By virtue of such arrangement and alignment of respective sheaths 126, holes 138, 140, and ultraviolet light sources 124, the ultraviolet light sources 124 may be easily inserted into and removed from the sheaths 126 as necessary for assembly, disassembly, and/or maintenance.

In accordance with the exemplary embodiments of the present invention, the sheaths 126 and ultraviolet light sources 124 are arranged in a single row with the centerlines 142, 144 of the sheaths 126 and ultraviolet light sources 124 defining angles, α, with the primary direction 136 of air travel through the reactor bed 102. All of the angles, α, generally (but not necessarily) have substantially the same angular measure and such angular measure is typically between zero degrees (0°) and one hundred eighty degrees (180°). According to the first exemplary embodiment depicted by FIGS. 1 and 2, the angles, α, have an angular measure of approximately ninety degrees (90°) such that the primary direction of 136 of air travel through the reactor bed 102 is substantially perpendicular to the longitudinal centerlines 142, 144 of the sheaths 126 and ultraviolet light sources 124. By virtue of the primary direction 136 of air flow through the reactor bed 102 being substantially transverse to (and, in the first exemplary embodiment, substantially perpendicular to) the longitudinal centerlines 142, 144 of the sheaths 126 and ultraviolet light sources 124 in the first exemplary embodiment, the sheaths 126 and ultraviolet light sources 124 act as obstructions, or baffles, to the flow of air through the reactor bed 102, create air turbulence within the reactor bed 102, and cause portions of the air attempting to flow through the reactor bed 102 in the primary direction 136 to be diverted into secondary directions (e.g., indicated by arrows 150) (see FIG. 2). By diverting portions of the air traveling through the reactor bed 102 into secondary directions 150, the residence time of such air portions in the reactor bed 102 is dramatically increased and, consequentially, there is a substantial increase in the number of bacteria, viruses, mold, fungi, spores, mycotoxins, allergens, other similar microorganisms or agents, and volatile organic compounds (VOCs) present in such air portions that come into contact with and are oxidized by hydroxyl radicals (OH⁻) produced by the photocatalytic reaction and adhering to the photocatalyst coated media 122.

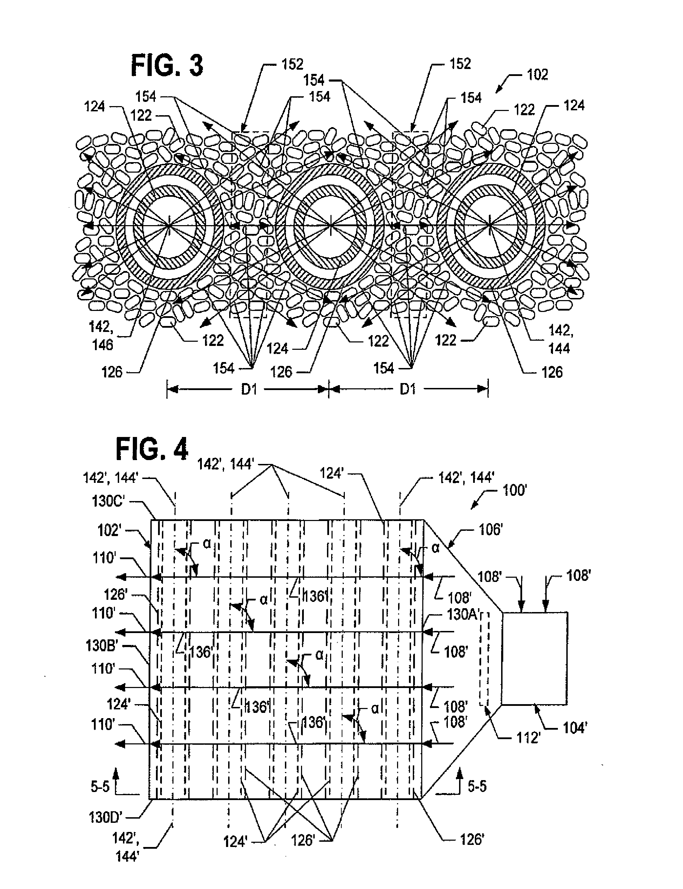

As illustrated in the schematic, partial sectional view of FIG. 3, the sheaths 126 of the reactor bed 102 are generally positioned adjacent to one another such that the corresponding ultraviolet light sources 124 therein are also generally positioned adjacent to one another and define a center-to-center distance, D1, therebetween having a measure in the range of about 1.25 inches to about 6 inches. In such an arrangement, portions of the photocatalyst coated media 122 reside between adjacent sheaths 126 and, hence, between adjacent ultraviolet light sources 124. Further, many of such photocatalyst coated media 122 reside within an elongate volume 152 extending between adjacent ultraviolet light sources 124 and panels 130C, 130D in which the irradiance of the ultraviolet light 154 striking the members 122 therein corresponds to the combined irradiance of the ultraviolet light 154 emitted outwardly by the adjacent ultraviolet light sources 124. Because the irradiance of the ultraviolet light 154 striking the photocatalyst coated media 122 within such elongate volumes 152 is higher than the irradiance of the ultraviolet light 154 striking photocatalyst coated media 122 not within such elongate volumes 152, the number of hydroxyl radicals (OH⁻) created by the photocatalytic reaction is greater within such elongate volumes 152 and, hence, the number of bacteria, viruses, mold, fungi, spores, mycotoxins, allergens, other similar microorganisms or agents, and volatile organic compounds (VOCs) present in air passing through such elongate volumes 152 that come into contact with and undergo an oxidation reaction with hydroxyl radicals (OH⁻) is substantially greater. As a consequence, each such elongate volume 152 is sometimes referred to as a "killing zone". Generally, in the exemplary embodiments described herein, each ultraviolet light source 124 is operable to produce ultraviolet light 154 having an irradiance greater than 5µW/cm² and preferably in a range of approximately 20µW/cm² to 30µW/cm². Thus, the irradiance of the ultraviolet light 154 striking the photocatalyst coated media 122 within such elongate volumes 152 is, typically, within a range of approximately 20µW/cm² to 60µW/cm². It should be noted that the number of hydroxyl radicals (OH⁻) created by the photocatalytic reaction is proportional to the irradiance of the ultraviolet light 154 striking the photocatalyst coated media 122 within such elongate volumes 152. Additionally, it should be noted that bacteria, viruses, mold, fungi, spores, mycotoxins, allergens, other similar microorganisms or agents may also be killed or rendered harmless simply by exposure to ultraviolet light from the ultraviolet light sources 124.

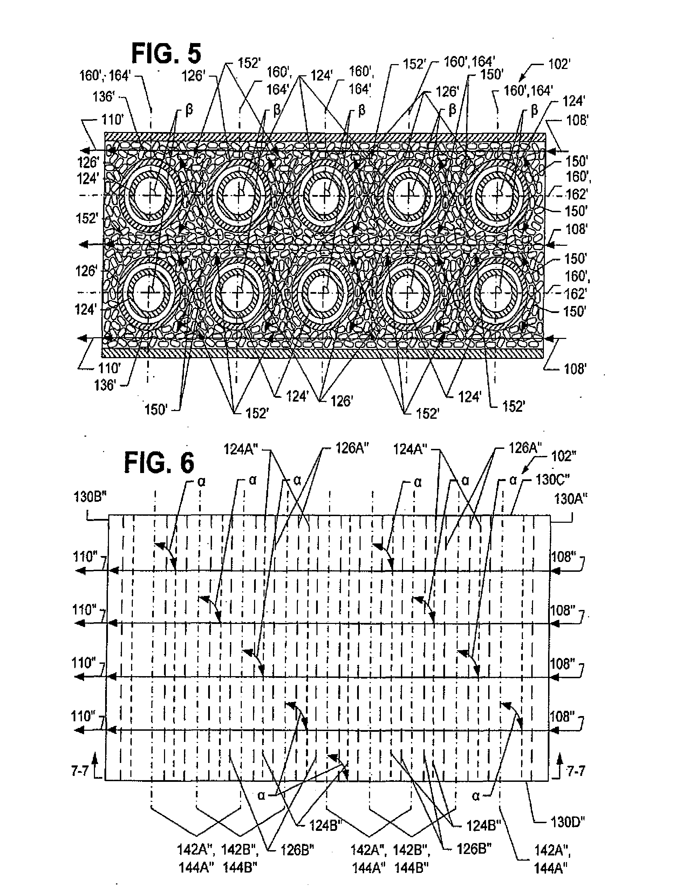

FIG. 4 displays a schematic, top plan view of a photocatalytic air treatment system 100' in accordance with a second exemplary embodiment of the present invention that is substantially similar to the first exemplary embodiment described herein. However, in the second exemplary embodiment, the plurality of ultraviolet light sources 124' and corresponding plurality of sheaths 126' are arranged within the reactor bed 102' in a row and column matrix 160' having multiple rows 162' and multiple columns 164'. The row and column matrix 160' is more clearly illustrated in the schematic, sectional view of FIG. 5. As illustrated, the rows 162' of ultraviolet light sources 124' and corresponding sheaths 126' define angles, β, with the columns 164' of ultraviolet light sources 124' and corresponding sheaths 126'. Generally, all of the angles, β, have the same angular measure. Also generally, each angle, β, has an angular measure of ninety degrees (90°). It should be noted, however, that in other exemplary embodiments of the present invention, angles, β, may have the same or different angular measures and/or angular measures other than ninety degrees (90°).

In a manner similar to that of the reactor bed 102 of the first exemplary embodiment, the primary direction 136' of air travel through the reactor bed 102' is transverse to the longitudinal centerlines 142', 144' of the respective sheaths 126' and ultraviolet light sources 124'. Notably, however, the row and column matrix 160' of ultraviolet light sources 124' and sheaths 126' of the second exemplary embodiment advantageously produces an increased number of obstructions, or baffles, to the flow of air through the reactor bed 102' than are present in the reactor bed 102 of the photocatalytic air treatment system 100 of the first exemplary embodiment. The row and column matrix 160' also advantageously results in the reactor bed 102' of the photocatalytic air treatment system 100' having an increased number of adjacent ultraviolet light sources 124' and an increased number of elongate volumes 152' extending between such adjacent ultraviolet light sources 124' and panels 130C', 130D' in which the irradiance of the ultraviolet light striking the photocatalyst coated media 122' therein corresponds to the combined irradiance of the ultraviolet light emitted outwardly by such adjacent ultraviolet light sources 124'. Due at least in part to the increased number of such elongate volumes 152", the arrangement of the ultraviolet light sources 124' of the photocatalytic air treatment system 100' of the second exemplary embodiment increases the number of bacteria, viruses, mold, fungi, spores, mycotoxins, allergens, other similar microorganisms or agents, and volatile organic compounds (VOCs) present in untreated air 108' that come into contact with and undergo an oxidation reaction with hydroxyl radicals (OH⁻), thereby increasing the number of the same that are killed, minerialized, and/or oxidized, as the case may be.

FIG. 6 displays a schematic, top plan view of a photocatalytic air treatment system 100" in accordance with a third exemplary embodiment of the present invention that is substantially similar to the second exemplary embodiment described herein. Similar to the photocatalytic air treatment system 100' of the second exemplary embodiment, the plurality of ultraviolet light sources 124" and corresponding plurality of sheaths 126" are arranged within the reactor bed 102" in multiple rows 162" and multiple columns 164" and the primary direction 136" of air flow through the reactor bed 102" is transverse to the longitudinal centerlines 142", 144" of the respective sheaths 126" and ultraviolet light sources 124". Also similarly, the longitudinal centerlines 142", 144" of the sheaths 126" and ultraviolet light sources 124" define angles, α, with the primary direction 136 of air flow through the reactor bed 102". Generally, the angles, α, have a measure of approximately ninety degrees (90°).

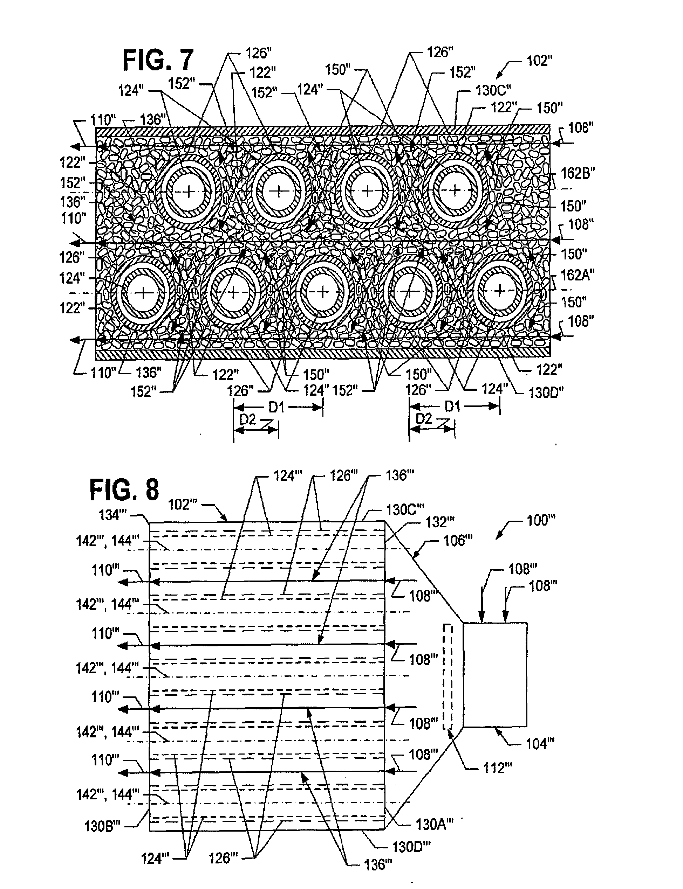

However, in the third exemplary embodiment and as is more clearly illustrated in the schematic, sectional view of FIG. 7, the ultraviolet light sources 124" and sheaths 126" of the second row 162B" are offset relative to the ultraviolet light sources 124" and sheaths 126" of the first row 162A" by an offset distance, D2. Generally, the offset distance, D2, has a measure of approximately one-half of the center-to-center distance, D1, between the ultraviolet light sources 124" and sheaths 126" of the first row 162A". By offsetting the ultraviolet light sources 124" and sheaths 126" of the second row 162B", the level of turbulence in the air flowing through the reactor bed 102" is increased with more portions of the air traveling in secondary directions 150". Perhaps more importantly, the nearer adjacency of the ultraviolet light sources 124" of the second row 162B" to multiple ultraviolet light sources 124" of the first row 162A" produces an increased number of elongate volumes 152" between such ultraviolet light sources 124" and within the reactor bed 102" in which the irradiance of the ultraviolet light striking the photocatalyst coated media 122" therein corresponds to the combined irradiance of the ultraviolet light emitted outwardly by such adjacent ultraviolet light sources 124". Because the arrangement of the ultraviolet light sources 124" increases the number of such elongate volumes 152" present within the reactor bed 102", the arrangement also increases the number of bacteria, viruses, mold, fungi, spores, mycotoxins, allergens, other similar microorganisms or agents, and volatile organic compounds (VOCs) present, in untreated air 108" that come into contact with and undergo an oxidation reaction with hydroxyl radicals (OH⁻), thereby killing them, mineralizing them, and/or oxidizing them.

FIG. 8 depicts a schematic, top plan view of a photocatalytic air treatment system 100"' for treating air according to a fourth exemplary embodiment of the present invention. As seen by viewing FIG. 8, the photocatalytic air treatment system 100'" comprises components substantially similar to those of the first exemplary embodiment described above. Thus, the photocatalytic air treatment system 100"' comprises a reactor bed 102"', an air-handling unit 104"', and a transition member 106"' coupled between the reactor bed 102"' and air-handling unit 104"' for guiding the flow of untreated air 108"' from the air-handling unit 104"' into the reactor bed 102"'. Similar to the reactor bed 102 of the first exemplary embodiment, the reactor bed 102"' of the fourth exemplary embodiment includes a plurality of sheaths 126"' for receiving a corresponding plurality of ultraviolet light sources 124"' therein and for separating the ultraviolet light sources 124"' from the photocatalyst coated media 122"' that substantially surround the sheaths 126"' and ultraviolet light sources 124"'. However, in the fourth exemplary embodiment, the longitudinal centerlines 142", 144" of the sheaths 126"' and ultraviolet light sources 124"' are substantially parallel to the primary direction 136"' of air flow through the reactor bed 102"'. As a consequence, a predominant portion of the untreated air 108."' entering the reactor bed 102"' travels through at least one elongate volume 152"' located between adjacent ultraviolet light sources 124"' and, therefore, a substantial number of bacteria, viruses, mold, fungi, spores, mycotoxins, allergens, other similar microorganisms or agents, and volatile organic compounds (VOCs) present in untreated air 108" come into contact with and are killed, mineralized, and/or oxidized by hydroxyl radicals (OH⁻).

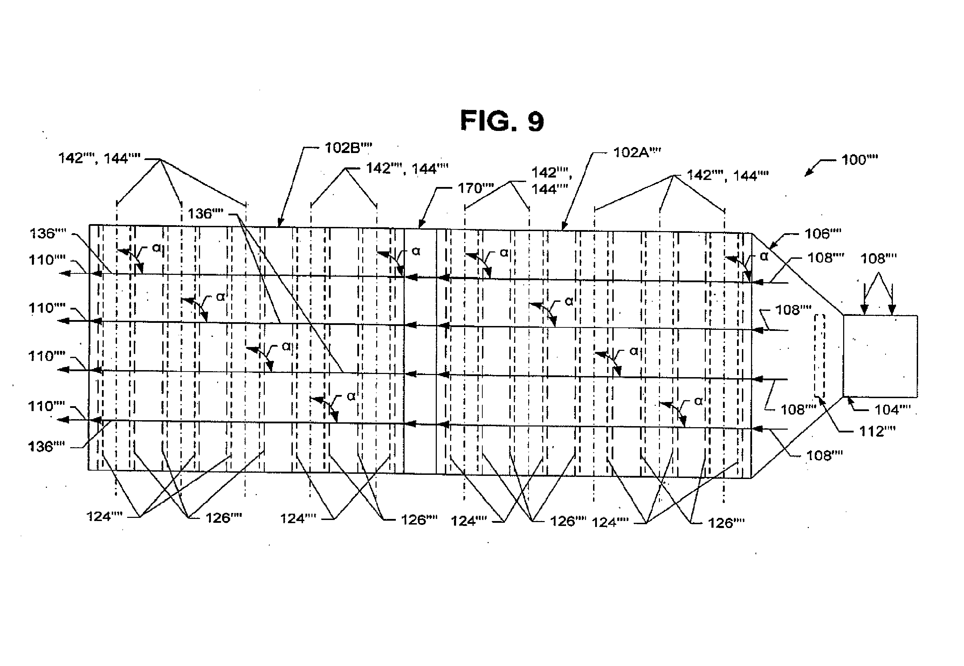

FIG. 9 displays a schematic, top plan view of a photocatalytic air treatment system 100"" in accordance with a fifth exemplary embodiment of the present invention. The photocatalytic air treatment system 100"" is substantially similar to that of the first exemplary embodiment, but includes multiple stages of air treatment instead of a single stage of air treatment as in the photocatalytic air treatment system 100 of the first exemplary embodiment. Due at least in part to the use of multiple stages of air treatment, the photocatalytic air treatment system 100"" of the fifth exemplary embodiment kills, mineralizes, and/or oxidizes larger numbers of bacteria, viruses, mold, fungi, spores, mycotoxins, allergens, other similar microorganisms or agents, and volatile organic compounds (VOCs) than the photocatalytic air treatment system 100 of the first exemplary embodiment.

In addition to similar components of the photocatalytic air treatment system 100 of the first exemplary embodiment, the photocatalytic air treatment system 100"" of the fifth exemplary embodiment comprises a first reactor bed 102A"" that is adapted to perform a first stage of air treatment and a second reactor bed 102B"" that is adapted to perform a second stage of air treatment. The first reactor bed 102A"" and second reactor bed 102B"" are connected by a coupling member 170"" for directing treated air 110A"" from the first reactor bed 102A"" into the second reactor bed 102B"" for further treatment. Generally, coupling member 170"" includes a plenum, duct, or other similar apparatus. Alternatively, in other exemplary embodiments, the first reactor bed 102A"" is abutted and directly connected to the second reactor bed 102B"" absent coupling member 170"".

In the photocatalytic air treatment system 100"", the first and second reactor beds 102A"", 102B"" each comprise a plurality of ultraviolet light sources 124"" and a corresponding plurality of sheaths 126"" having respective longitudinal centerlines 144"", 142"" that define angles, α, relative to the primary direction 136"" of air flow through the reactor beds 102"". Generally, each angle, α, has the same angular measure. Also generally, each angle, α, has an angular measure within a range of forty-five degrees (45°) to one hundred thirty-five degrees (135°). Thus, the ultraviolet light sources 124"" and sheaths 126"" are oriented such that the primary direction 136"" of air flow through each reactor bed 102"" is substantially transverse to the longitudinal centerlines 144"", 142"" of the ultraviolet light sources 124"" and sheaths 126"", thereby creating air turbulence within the reactor beds 102"", causing air to flow in secondary directions 150"" within the reactor beds 102"", increasing the residence time for bacteria, viruses, mold, fungi, spores, mycotoxins, allergens, other similar microorganisms or agents, and volatile organic compounds (VOCs) within the reactor beds 102"", and increasing the number of the same that are killed, rendered non-reproducible, mineralized, and/or oxidized.

It should be noted that although the angular measure of angles, α, is generally the same in both reactor beds 102"" of the photocatalytic air treatment system 100"", the scope of the present invention includes other exemplary embodiments in which the angular measure of angles, α, is not the same. Therefore, the scope of the present invention includes photocatalytic air treatment systems 100"" in which the primary direction 136"" of the flow of air through one reactor bed 102"" is substantially transverse to the longitudinal centerlines 144"", 142"" of the ultraviolet light sources 124"" and sheaths 126"", while the primary direction 136"" of the flow of air through a second reactor bed 102"" is substantially parallel to the longitudinal centerlines 144"", 142"" of the ultraviolet light sources 124"" and sheaths 126"". Further, the scope of the present invention includes photocatalytic air treatment systems 100"" in which the primary direction 136"" of the flow of air through both reactor beds 102"" is substantially parallel to the longitudinal centerlines 144"", 142"" of the ultraviolet light sources 124"" and sheaths 126"".

Before proceeding with a description of a method of operation of the photocatalytic air treatment systems of the exemplary embodiments, it should also be noted that in other exemplary embodiments of the present invention, at least one pair of the ultraviolet light sources thereof have a distance therebetween that is different from the distance between the ultraviolet light sources of other pairs of ultraviolet light sources of the plurality of ultraviolet light sources. Additionally, it should be noted that in other exemplary embodiments of the present invention, the plurality of ultraviolet light sources are arranged in different arrangements such that the time required for moving air to travel or pass by all of the ultraviolet light sources is increased relative to the time required for moving air to travel or pass by all of the ultraviolet light sources of an arrangement thereof in which the plurality of ultraviolet light sources are positioned substantially in a single row or single column. By virtue of arranging the plurality of ultraviolet light sources in an arrangement that causes such an increase in the time required for air to travel or pass by all of the ultraviolet light sources, the number of collisions (or contacts) between bacteria, viruses, mold, fungi, spores, mycotoxins, allergens, other similar microorganisms or agents, and/or volatile organic compounds (VOCs) present in the air and the plurality of media of a reactor bed are also increased, thereby resulting in an increase in the number of bacteria, viruses, mold, fungi, spores, mycotoxins, allergens, other similar microorganisms or agents, and/or volatile organic compounds (VOCs) that are killed, rendered non-reproducible, mineralized, and/or oxidized.

In operation, the photocatalytic air treatment systems 100, 100', 100", 100"', 100"" of the various exemplary embodiments function according to substantially the same method. Therefore, although the following description is directed primarily to the photocatalytic air treatment system 100 of the first exemplary embodiment, it is generally applicable to the other exemplary embodiments as well. The photocatalytic air treatment system 100 is generally positioned within the environment in which air is to be treated and is connected to an appropriate electrical power supply. Once activated, the air-handling unit 104 pulls in untreated air 108 from the environment through its air intake and blows the untreated air 108 out through its exhaust and into the transition member 106 at an appropriate static pressure, velocity, and volumetric flow rate. While traveling though the transition member 106, the untreated air 108 may be heated by heating element 112 in order to raise the temperature and reduce the relative humidity of the untreated air 108. By increasing the air's temperature and reducing its relative humidity, the reaction rates of the photocatalytic and oxidation reactions occurring within the reactor bed 102 are increased, thereby resulting in an increased production of hydroxyl radicals (OH⁻) and an increased number of bacteria, viruses, mold, fungi, spores, mycotoxins, allergens, other similar microorganisms or agents, and volatile organic compounds (VOCs) being killed, mineralized, and/or oxidized, as the case may be.

The transition member 106 then directs the untreated air 108 through a HEPA filter (if one is present) and into the reactor bed 102 through the reactor bed's air inlet 132. Once inside the reactor bed 102, the untreated air 108 flows through the photocatalyst coated media 122 and toward the reactor bed's air outlet 134 in the primary direction 136 of air flow. However, as the untreated air 108 comes into contact with the sheaths 126, portions of the untreated air 108 are deflected and redirected in secondary directions 150 and into the elongate volumes 152 of photocatalyst coated media 122 located between adjacent ultraviolet light sources 124. In the elongate volumes 152, the untreated air 108 comes into contact with photocatalyst coated media 122 that has been exposed to ultraviolet light having an irradiance corresponding to the combined irradiance of the ultraviolet light 154 emitted outwardly by the adjacent ultraviolet light sources 124. Due at least in part to the photocatalyst coated media 122 of the elongate volumes 152 being exposed to a greater irradiance of ultraviolet light 154, the photocatalyst coated media 122 therein host a greater number of hydroxyl radicals (OH⁻) to which bacteria, viruses, mold, fungi, spores, mycotoxins, allergens, other similar microorganisms or agents, and volatile organic compounds (VOCs) come into contact. Upon their contact, the hydroxyl radicals (OH⁻) react in an oxidation reaction with the bacteria, viruses, mold, fungi, spores, mycotoxins, allergens, other similar microorganisms or agents, and volatile organic compounds (VOCs), causing them to be killed, rendered non-reproducible, oxidized, and/or mineralized, as the case may be, and improving the quality of the untreated air 108. It should be noted that the portions of the untreated air 108 that do not flow through an elongate volume 152 are also subjected to hydroxyl radicals (OH⁻) present on photocatalyst coated media 122 with similar results, but they are not subjected to the same highly concentrated numbers of hydroxyl radicals (OH⁻) that are present within the elongate volumes 152. It should also be noted that the longer untreated air 108 is resident within the reactor bed 102, there is an increased likelihood that the bacteria, viruses, mold, fungi, spores, mycotoxins, allergens, other similar microorganisms or agents, and volatile organic compounds (VOCs) therein will come into contact with hydroxyl radicals (OH⁻) and be killed, rendered non-reproducible, oxidized, and/or mineralized, thereby improving the quality of the treated air 110.

After flowing through the photocatalyst coated media 122, treated air 110 exits the reactor bed 102 through the reactor bed's outlet 134 and back into the environment of the photocatalytic air treatment system 100. Of course, if the photocatalytic air treatment system 100 has multiple stages of air treatment, the untreated air 108 flows through multiple reactor beds 102 before being reintroduced into the environment, thereby resulting in increased air treatment.

The photocatalytic air treatment system 100 of the present invention may be utilized to treat air as described herein in a variety of applications. For example, in certain implementations, the photocatalytic air treatment system 100 or a variant thereof may be integrated into or used in connection with residential and commercial grade refrigerators, refrigeration systems, and wine coolers to treat the air present within refrigerated environments thereof, supplied thereto, and/or exhausted therefrom. In other implementations, the photocatalytic air treatment system 100 or a variant thereof may be incorporated into or utilized in conjunction with central and standalone air conditioning systems for residential and/or commercial use that may or may not include additional air handling units in order to treat the conditioned air supplied to residential and commercial rooms, buildings, facilities, and/or structures. In still other implementations, the photocatalytic air treatment system 100 or a variant thereof may be integrated into or used in connection with bathroom and/or vehicle air delivery, air exhaust, and/or air conditioning systems to treat air provided to and/or exhausted from bathrooms and/or the passenger compartments of vehicles and other mobile equipment. In still other implementations, the photocatalytic air treatment system 100 or a variant thereof may be incorporated into, attached to, or used with an air sanitizing module for an anesthesia machine to remove trace volatile organic compounds (VOCs) generated by anesthetic agents. In still other implementations, the photocatalytic air treatment system 100 or a variant thereof may be designed and integrated into an existing portable air purifying device used in hospitals. In still other implementations, the photocatalytic air treatment system 100 or a variant thereof may be incorporated into a ceiling or whole house fan for use in various rooms or structures, including infants' rooms. In still other implementations, the photocatalytic air treatment system 100 may be incorporated into or utilized with a hospital bed to provide onboard air treatment and/or purification. In yet other implementations, the photocatalytic air treatment system 100 or a variant thereof may be incorporated into or utilized in connection with cages or other enclosures used to house and/or transport animals to treat air supplied thereto and/or exhausted therefrom.

Whereas the present invention has been described in detail above with respect to exemplary embodiments thereof, it should be understood that variations and modifications might be effected within the scope of the present invention, as described herein before and as defined in the appended Claims.

## Claims

1. An apparatus for treating air using a photocatalytic reaction, said apparatus comprising;
a reactor bed including a plurality of media coated at least partially with a photocatalyst substance; and
a plurality of ultraviolet light sources immersed within and substantially surrounded by said plurality of media, wherein a portion of said plurality of media resides substantially between ultraviolet light sources of said plurality of ultraviolet light sources for receiving ultraviolet light from said ultraviolet light sources having an irradiance greater than 5µW/cm² and for producing a plurality of hydroxyl radicals from a photocatalytic reaction of said photocatalyst substance induced by said ultraviolet light; and
an air-handling unit in fluid communication with said reactor bed and adapted to cause air to flow by and between media of said portion of said plurality of media, said air handling unit including a transition member configured to direct and distribute untreated air exiting the air handling unit into the reactor bed to provide a substantially equal flow of untreated air to all parts of the reactor bed.

2. An apparatus for treating air using a photocatalytic reaction, said apparatus comprising;
a reactor bed including a plurality of media coated at least partially with a photocatalyst substance; and
a plurality of ultraviolet light sources immersed within and substantially surrounded by said plurality of media, wherein a portion of said plurality of media resides substantially between ultraviolet light sources of said plurality of ultraviolet light sources for receiving ultraviolet light from said ultraviolet light sources having an irradiance greater than 5µW/cm² and for producing a plurality of hydroxyl radicals from a photocatalytic reaction of said photocatalyst substance induced by said ultraviolet light; and
an air-handling unit in fluid communication with said reactor bed and adapted to cause air to flow by and between media of said portion of said plurality of media; and a heating element adapted to heat the untreated air prior to entrance into the reactor bed.

3. The apparatus of Claim 1 or Claim 2, wherein the apparatus is for treating air of a refrigerated environment and said refrigerated environment comprises one of the following group:
a refrigerated cavity of a refrigerator;
a refrigerated cavity of a wine cooler; and
a refrigerated cavity of a refrigeration system.

4. The apparatus of any one of the preceding claims, wherein at least one ultraviolet light source of said plurality of ultraviolet light sources has a longitudinal axis extending in a first direction and is oriented such that air flows relative to said one ultraviolet light source predominantly in a second direction substantially transverse to said first direction.

5. The apparatus of any one of the preceding claims, wherein at least one ultraviolet light source of said plurality of ultraviolet light sources has a longitudinal axis extending in a first direction and is oriented such that air flows relative to said one ultraviolet light source predominantly in a second direction substantially parallel to said first direction.

6. The apparatus of any one of the preceding claims, wherein said plurality of ultraviolet light sources are positioned in an arrangement in which the time required for moving air to travel by all of said ultraviolet light sources of said plurality of ultraviolet light sources is increased relative to the time required for moving air to travel by all of said ultraviolet light sources of said plurality of ultraviolet light sources in a different arrangement in which said plurality of ultraviolet light sources are positioned substantially in a single row or single column.

7. The apparatus of any one of the preceding claims, wherein said portion of said plurality of media comprises a first portion of said plurality of media and a pair of ultraviolet light sources of said plurality of ultraviolet light sources comprises a first pair of ultraviolet light sources of said plurality of ultraviolet light sources, and wherein a second portion of said plurality of media resides substantially between a second pair of ultraviolet light sources of said plurality of ultraviolet light sources for receiving ultraviolet light from said second pair of ultraviolet light sources having a combined irradiance in a range of 20 µW/cm² to 60 µW/cm².

8. The apparatus of any one of the preceding claims, wherein said apparatus further comprises a sheath in contact with at least one media of said plurality of media and interposed between said one media and at least one ultraviolet light source of said plurality of ultraviolet light sources; optionally wherein said sheath is formed from a quartz material.

9. The apparatus of Claim 8, wherein a substantial portion of said one ultraviolet light source resides within said sheath; optionally wherein said substantial portion of said one ultraviolet light source is removable from within said sheath.

10. The apparatus of any one of the preceding claims, wherein media of said plurality of media are formed from a material that induces said photocatalyst substance to form in a nano-particle structure thereon; optionally wherein said material comprises bora silica.

11. The apparatus of any one of the preceding claims, wherein said plurality of media are further coated at least partially with an enhancing substance adapted to enhance the reaction rate of said photocatalytic reaction; optionally wherein said enhancing substance comprises zirconium dioxide.

12. The apparatus of any one of the preceding claims, wherein said photocatalyst substance comprises titanium dioxide.

13. The apparatus of any one of the preceding claims, wherein adjacent ultraviolet light sources of said plurality of ultraviolet light sources are arranged having a center-to-center distance therebetween within a range of 1.25 inches and 6 inches; optionally wherein ultraviolet light from said adjacent ultraviolet light sources has at least one wavelength in the range of 1 nanometer to 399 nanometers.

14. The apparatus according to Claim 1 or Claim 2, wherein the plurality of media include generally tubular media.

15. The apparatus according to Claim 1 or Claim 2, wherein the plurality of ultraviolet light sources are arranged in a row and column matrix having multiple rows and multiple columns.

16. A method for treating air using a photocatalytic reaction, the method comprising the steps of: positioning a plurality of media coated at least partially with a photocatalyst substance substantially between a plurality of adjacent ultraviolet light sources; irradiating the plurality of media with ultraviolet light from the plurality of adjacent ultraviolet light sources to produce a volume of media of the plurality of media in which the combined irradiance of the ultraviolet light impinging thereon is greater than 5 µW/cm² and to produce a plurality of hydroxyl radicals from a photocatalytic reaction of the photocatalyst substance; moving air having at least one undesired element entrained therein by the plurality of adjacent ultraviolet light sources and by the media for reaction of at least one hydroxyl radical of the plurality of hydroxyl radicals with the at least one undesired element in an oxidation reaction; and delivering the air to an environment.

17. The method of Claim 16, wherein the air is delivered to a refrigerated environment, said refrigerated environment comprising one of the following group:
a refrigerated cavity of a refrigerator;
a refrigerated cavity of a wine cooler; and
a refrigerated cavity of a refrigeration system.

18. The method of any one of Claims 16 or 17, wherein the method further comprises a step of enclosing at least one of the adjacent ultraviolet light sources at least partially within an enclosure to reside between the at least one adjacent ultraviolet light source and the plurality of media.

19. The method of any one of Claims 16 to 18, wherein the method further comprises a step of arranging the plurality of adjacent ultraviolet light sources at respective locations to provide center-to-center distance therebetween within a range of 1.25 inches and 6 inches.

20. The method of any one of Claims 16 to 19, wherein the method further comprises a step of arranging the plurality of adjacent ultraviolet light sources in an arrangement that produces multiple volumes of media of the plurality of media in which the combined irradiance of the ultraviolet light impinging thereon is within a range of 20 µW/cm² to 60 µW/cm²_{.}
